(19) Europäisches Patentamt
European Patent Office
Office européen des brevets

(11) Veröffentlichungsnummer: **0 058 333**
**B1**

(12)
# EUROPÄISCHE PATENTSCHRIFT

(45) Veröffentlichungstag der Patentschrift: **20.06.84**

(51) Int. Cl.³: **C 07 C 120/10,** C 07 C 121/16 // A01N43/64

(21) Anmeldenummer: **82100697.0**

(22) Anmeldetag: **02.02.82**

(54) **Verfahren zur Herstellung von Nitrilen aus Formamiden.**

(30) Priorität: **14.02.81 DE 3105452**

(43) Veröffentlichungstag der Anmeldung: **25.08.82 Patentblatt 82/34**

(45) Bekanntmachung des Hinweises auf die Patenterteilung: **20.06.84 Patentblatt 84/25**

(84) Benannte Vertragsstaaten: **BE CH DE FR GB IT LI NL**

(56) Entgegenhaltungen:
**EP - A - 0 020 950**
**DE - A - 2 706 863**
**DE - B - 1 068 241**

**Chemical Abstracts Band 72, Nr. 1, 5. Januar 1970, Columbus, Ohio, USA, M. GREENHALGH et al. "Purines, pyrimidines, and imidazoles. XXXII. Synthesis of 5-amino-4-cyano-1-(Beta-D-ribofuranosyl)imidazole 5'-phosphate and a new mild method for dehydration of carboxamides to nitriles" Seiten 348, 349, Abstract Nr. 3701b**

(73) Patentinhaber: **BAYER AG, Konzernverwaltung RP Patentabteilung, D-5090 Leverkusen 1 Bayerwerk (DE)**

(72) Erfinder: **Arlt, Dieter, Prof. Dr., Rybnikerstrasse 2, D-5000 Köln 80 (DE)**
Erfinder: **Klein, Gerhard, Dr., v.-Flotow-Strasse 7, D-4019 Monheim (DE)**

**EP 0 058 333 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft ein neues, nicht-katalytisches Verfahren zur Herstellung von Carbonsäurenitrilen mit einem tertiären alpha-C-Atom, durch Dehydratisierung und Isomerisierung aus den entsprechenden Formamiden.

Es ist bekannt, N-substituierte Formamide an speziellen Katalysatoren bei hohen Temperaturen zu Nitrilen und Wasser umzusetzen (vgl. z.B. DE-OS'sen 1 908 967, 2 036 503, 2 706 863, 2 827 058, EP-A- 0 020 950).

Dabei muss in vielen Fällen zur Erzielung einer befriedigenden Ausbeute die Rekation bei geringem Umsatz durchgeführt werden. Diese Verfahrensweise erfordert die Rückführung des Einsatzproduktes und ergibt nur eine geringe Raum-Zeit-Ausbeute. Ein weiterer erheblicher Nachteil dieser bekannten katalytischen Verfahren besteht darin, dass eine Anzahl von Formamiden überhaupt nur sehr unbefriedigende Ausbeuten an erwünschtem Nitril ergibt und dass die Aktivität des Katalysators nach kurzer Zeit nachlässt. In solchen Fällen ist diese bekannte Verfahrensweise technisch nicht durchführbar. Insbesondere sind die Ausbeuten und die Standzeiten der beschriebenen Katalysatoren für ein technisches Herstellungsverfahren nicht ausreichend, wenn Formamide der Formel R-NHCHO eingesetzt werden, deren Rest R ein über ein tertiäres Kohlenstoffatom an das Stickstoffatom gebundener Alkylrest ist (vgl. z.B. die Selektivitätsangabe in Beispiel 3 der DE-OS 2 706 863). Solche Formamide neigen bei höheren Temperaturen nicht nur wie Formamide allgemein zur Decarbonylierung sondern auch zu Eliminierung des Restes R unter Bildung von Olefin.

Es wurde nun überraschend gefunden, dass man Nitrile mit einem tertiären alpha-C-Atom der allgemeinen Formel R-CN (I) aus N-substituerten Formamiden der allgemeinen Formel

R-NH-CHO (II),

in welchen

R für einen gesättigten oder ungesättigten aliphatischen Kohlenwasserstoffrest mit 4 bis 17 Kohlenstoffatomen steht, der ein in (II) direkt an die Formamidgruppe bzw. in (I) direkt an die Nitrilgruppe gebundenes tertiäres Kohlenstoffatom enthält,

in einfacher Weise mit guten Ausbeuten und mit guter Selektivität herstellen kann, indem man die N-substituierten Formamide (II) mit der mindestens stöchiometrischen Menge eines Carbonsäureanhydrids und/oder Keten in der Gasphase bei Temperaturen oberhalb 250°C ohne Verwendung eines Katalysators umsetzt.

Das erfindungsgemässe Verfahren erlaubt erstmals auch solche Nitrile aus den entsprechenden Formamiden mit hoher Selektivität herzustellen, deren Nitrilgruppe direkt an tertiäre Kohlenstoffatome gebunden ist. Dabei verzichtet das erfindungsgemässe Verfahren auf die Verwendung von Katalysatoren und vermeidet damit ausserdem die durch geringe Standzeit und begrenzten Umsatz bedingten Mängel der vorbekannten katalytischen Verfahren.

Die nach dem erfindungsgemässen Verfahren erreichten Selektivitäten und Ausbeuten sind besonders überraschend, da es bekannt ist (vgl. J. Org. Chem. 33 (1968), S. 4050-4054), dass die Pyrolyse von N-Formylacetamiden nur in sehr schlechten Ausbeuten zu Nitrilen führt. N-Formylcarbonsäureamide sind auch im Reaktionsgemisch des erfindungsgemässen Verfahrens als Zwischenstufen nachweisbar.

Verwendet man als Ausgangsstoff beispielsweise N-tert.-Butylformamid und als Carbonsäureanhydrid beispielsweise Acetanhydrid, so kann der Reaktionsablauf durch das folgende Formelschema wiedergegeben werden:

$$(CH_3)_3C{-}NH{-}CHO + (CH_3CO)_2O \longrightarrow$$

$$(CH_3)_3C{-}CN + 2\,CH_3COOH$$

Die als Ausgangsstoffe zu verwendenden N-substituierten Formamide sind durch die Formel (II) allgemein definiert. In dieser Formel steht R vorzugsweise für aliphatische Kohlenwasserstoffreste mit 4 bis 16 Kohlenstoffatomen, deren tertiäres Kohlenstoffatom direkt an die Formamidgruppe gebunden ist.

Als Ausgangsstoffe der Formel R-NHCHO (II) seien beispielsweise genannt: tert.-Buthylformamid, 1,1-Dimethylpropyl-formamid, 1,1-Dimethylpropargyl-formamid, 1-Methyl-pentyl-formamid.

Die erfindungsgemäss verwendbaren Formamide der Formel (II) sind bekannt oder können nach bekannten Methoden hergestellt werden, insbesondere durch die sogenannten Graf-Ritter-Reaktion aus Cyanwasserstoff und Olefinen (vgl. hierzu z.B. DE-PS 870 856; Methodicum Chimicum, Bd. 6 (1974), S. 710; Org. Reactions Vol. 17 (1969), S. 213 ff).

Bei dem erfindungsgemässen Verfahren werden Carbonsäureanhydride und/oder Keten eingesetzt. Als Carbonsäureanhydride kommen insbesondere Acetanhydrid und die Anhydride der Säuren, deren Nitril erzeugt werden soll (z.B. Pivalinsäureanhydrid, wenn Pivalonitril hergestellt werden soll), zur Anwendung. Das Carbonsäureanhydrid bzw. Keten wird wie schon angegeben mindestens stöchiometrisch zur eingesetzten Formamidmenge angewendet, vorteilhaft wird jedoch ein Überschuss im Molverhältnis 1,5 bis 3:1 eingesetzt. Als Reaktionsprodukt entsteht neben dem gewünschten Nitril die dem Carbonsäureanhydrid bzw. Keten zugrunde liegende Carbonsäure, die bei technischer Durchführung wieder in das verfahrensgemäss einzusetzende Carbonsäureanhydrid bzw. Keten zurücküberführt werden kann.

Zur Erzielung guter Selektivität ist es wichtig, eine Flüssigphasenreaktion zu vermeiden. Die Umsetzung wird im allgemeinen so durchgeführt, dass die Reaktionskomponenten gemeinsam oder getrennt verdampft und in eine Reaktionszone eingebracht werden, wobei das Reaktionsgemisch auf die erforderliche Temperatur erwärmt wird. Die notwendige Temperatur kann z.B. durch Aussenheizung erfolgen, es kann aber auch das Carbonsäureanhydrid bzw. Keten vor der Reaktionszone erhitzt und als Wärmeträger benutzt werden.

Die Reaktion kann mit oder ohne Inertgasverdünnung durchgeführt werden. Im allgemeinen arbeitet man im Temperaturbereich zwischen etwa 320 und 500°C. Es kann vorteilhaft sein, die Reaktion durch eine genaue Temperaturführung zu steuern. So wird eine sehr gute Selektivität an Nitrilen (I) dadurch erreicht, dass man die N-substituierten Formamide (II) zunächst bei Temperaturen von etwa 390–420°C zu acylierten Formamiden umsetzt, die dann in einem höheren Temperaturbereich von etwa 420–500°C in erwünschter Weise in Nitrile (I) und Carbonsäure fragmentieren.

Die Verdampfung und Reaktion der Ausgangskomponenten kann sowohl unter Normaldruck wie auch bei vermindertem Druck erfolgen. Übliche technisch benutzte Verdampfer können benutzt werden, z.B. Schlangenrohr- und Fallfilm-Verdampfer. Die Reaktion wird z.B. in Reaktionsrohren aus VA-Stahl durchgeführt. Das Verfahren kann auch im Fliess- oder Wirbelbett ausgeführt werden. Die Reaktionsprodukte werden nach Verlassen der Reaktionszone gekühlt und kondensiert. Eine Trennung des Stoffgemisches erfolgt im allgemeinen durch fraktionierte Vakuumdestillation. Bei der Aufarbeitung ist ein längeres Erwärmen der Flüssigphase auf Temperaturen über 100°C auszuschliessen, um eine Ausbeuteminderung zu vermeiden.

Die nach dem erfindungsgemässen Verfahren herstellbaren Nitrile (I) sind wertvolle Zwischenprodukte, z.B. zur Herstellung von Herbiziden, Tensiden und Korrosionsschutzmitteln.

So kann beispielsweise Pivalonitril (A) durch katalytische Hydrierung in Neopentylamin (B) überführt werden, welches auf verschiedenen Wegen zu dem bekannten herbiziden Wirkstoff 1-Amino-6-ethylthio-3-neopentyl-1,3,5-triazin-2,4(1H, 3H)-dion (K) (vgl. z.B. DK-PS 136 067) umgesetzt werden kann:

$$\underset{(A)}{(CH_3)_3C\text{-}CN} \xrightarrow[\text{[Kat.]}]{2\,H_2} \underset{(B)}{(CH)_3C\text{-}CH_2\text{-}NH_2}$$

Ein Verfahrensweg führt über folgende Stufen (vgl. BE-PS 682 820; Angew. Chem. 82 (1970), S. 63–67; Synthesis 1970, S. 542–543; DE-OS 2 254 200):

$$(B) \longrightarrow \underset{(C)}{(CH_3)_3C\text{-}CH_2\text{-}NH\text{-}CHO} + \underset{(D)}{2Cl\text{-}CO\text{-}S\text{-}Cl} \xrightarrow[-2HCl]{-COCl_2}$$

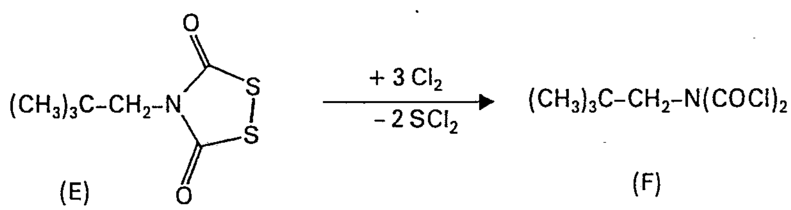


(E) (F)

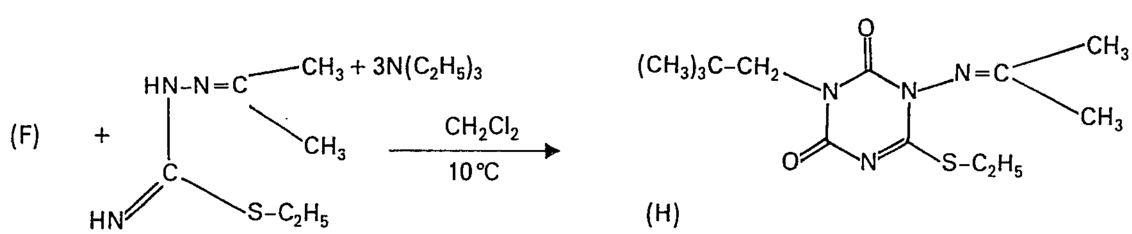


(F) (H)

xHBr (G)

[+2 $N(C_2H_5)_3 \cdot HCl$ + $N(C_2H_5)_3 \cdot HBr$]

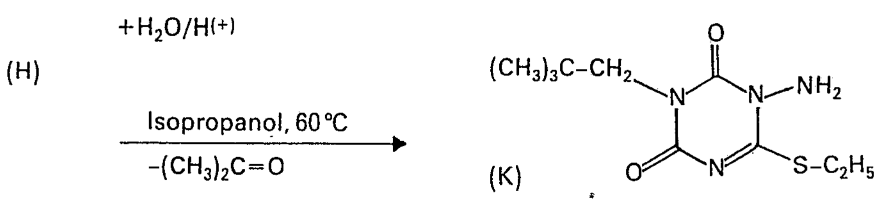


(H) (K)

Ein anderer Verfahrensweg führt über folgende Stufen (vgl. DE-OS 3 006 226 und DE-OS 3 006 263):

(B) $\longrightarrow$ $(CH_3)_3C-CH_2-N(CO-OC_6H_5)_2$ (L)

(L) + (G) $\longrightarrow$ (H) $\longrightarrow$ (K)

Die nachfolgenden Herstellungsbeispiele sollen zur weiteren Erläuterung der Erfindung dienen.

Herstellungsbeispiele

Beispiel 1

Herstellung von Pivalonitril, $(CH_3)_3C-CN$

Pro Stunde werden 23 g N-tert.-Butylformamid und 69 g Acetanhydrid getrennt in einen auf jeweils 220°C geheizten Vorverdampfer und die Gase in ein mit Raschigringen (Durchmesser 12 mm) gefülltes Quarzrohr (Querschnitt des Rohres 90 mm, Volumen 1180 ml), das elektrisch auf 420°C geheizt ist, eindosiert. Die Pyrolysegase werden in einer gekühlten Vorlage kondensiert; man erhält 91,7 g Kondensat pro Stunde. Die gaschromatographisch ermittelte Selektivität bezogen auf Pivalonitril beträgt 92% bei einem Umsatz von 88%.

Beispiel 2

Herstellung von Pivalonitril, $(CH_3)_3C-CN$

Pro Stunde werden 69 g eines Gemisches aus Pivalinsäureanhydrid und N-tert.-Butylformamid (Gewichtsverhältnis 2,8:1) in ein auf 420°C geheiztes V4A-Rohr (Querschnitt 8 mm, Volumen 800 ml) getropft. Bei einer Verweilzeit von 111 sec ergibt sich gaschromatographisch eine Selektivität von 93% bezogen auf Pivalonitril bei einem Umsatz von 94%. Die Laufzeit beträgt 122 Stunden.

Beispiel 3

a) Herstellung von N-(1,1-Dimethyl-propyl)-formamid,

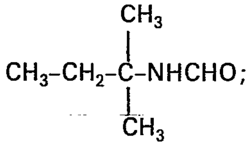

;

durch eine Graf-Ritter-Reaktion:

In eine Lösung von 250 ml Blausäure in 410 g Methansulfonsäure werden innerhalb einer Stunde gleichzeitig 210 g 2-Methyl-buten-(2) und 54 g Wasser eingetropft; dabei wird die Reaktionstemperatur durch Kühlung auf 20–40°C gehalten. Nach 4 Stunden wird die überschüssige Blausäure abdestilliert, der Rückstand mit Eis/Natronlauge neutralisiert und anschliessend das Formamid mit Methylenchlorid extrahiert. Die Methylenchloridlösung wird destillativ aufgearbeitet. Man erhält 245 g N-(1,1-Dimethyl-propyl)-formamid.

b) Herstellung von 2-Methyl-butyro-nitril-(2),

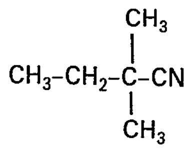

115 g N-(1,1-Dimethyl-propyl)-formamid und 300 g Acetanhydrid werden in eine Apparatur wie in Beispiel 1 beschrieben innerhalb von 5 Stunden eindosiert, wobei die Vorverdampfer-Temperatur 240°C, die durchschnittliche Temperatur im Rohrreaktor 430°C beträgt. Man erhält 402 g Pyrolysat, das nach gaschromatographischer Analyse 19,5% (80% Selektivität) 2-Methyl-butyro-nitril-(2) enthält.

Das Produkt wird durch fraktionierte Destillation isoliert (Siedepunkt 130–131°C).

Beispiel 4

Herstellung von Pivalonitril, $(CH_3)_3C-CN$

Pro Stunde werden 60 g eines Gemisches aus Acetanhydrid und N-tert.-Butylformamid (Gewichtsverhältnis 3:1) in ein elektrisch beheiztes (400°C) V4A-Rohr (Innendurchmesser 8 mm, Volumen 750 ml) getropft. Die Verweilzeit beträgt 83 sec. Das Pyrolysat wird in einem Produktkühler und einem Vorlagegefäss, das mit Kühlsole von −20°C gekühlt ist, weitgehend kondensiert. Nach 147 Stunden wird der Versuch beendet. Man erhält 8670 g Kondensat, aus dessen gaschromatographischer Analyse sich für Pivalonitril eine Selektivität von 84% bei einem Umsatz von 83% ergibt. Das Kondensat kann bei 300 mbar ohne Nebenreaktionen des nicht umgesetzten N-tert.-Butylformamids aufdestilliert werden.

Beispiel 5

Herstellung von Pivalonitril, $(CH_3)_3C-CN$

Pro Stunde werden 10 g N-tert.-Butylformamid und 19 g Keten in ein elektrisch beheiztes (400°C) V4A-Rohr (Innendurchmesser 6 mm, Volumen 90 ml) eindosiert. Die Verweilzeit beträgt 40 sec. Das Pyrolysat wird in einem auf −20°C gekühlten Vorlagegefäss kondensiert. Man erhält 22,0 g Kondensat, das nach gaschromatographischer Analyse 24,9% Pivalonitril enthält (Selektivität 76%, Umsatz 87%).

Beispiel 6

Pro Stunde werden 44 g N-tert.-Butylformamid und 108 g Acetanhydrid getrennt in einen auf jeweils 220°C geheizten Vorverdampfer getropft und die Gase in ein mit Raschigringen (Durchmesser 12 mm) gefülltes Quarzrohr (Querschnitt des Rohres 90 mm, Länge 300 mm) geleitet.

Das Reaktionsrohr wird im unteren Drittel auf 450°C, der obere Teil auf 420°C geheizt. Die Pyrolysegase werden in einer gekühlten Vorlage kondensiert. Man erhält 151 g Kondensat pro Stunde.

Das Kondensat enthält nach der gaschromatographischen Analyse 27.8 g Pivalonitril und 7.9 g nicht umgesetztes N-tert.-Butylformamid (Selektivität von 94% bei einem Umsatz von 82%). Die Laufzeit beträgt 500 Stunden.

## Patentansprüche

1. Verfahren zur Herstellung von Nitrilen mit einem tertiären alpha-C-Atom der allgemeinen Formel R-CN (I) aus N-substituierten Formamiden der allgemeinen Formel

R-NH-CHO (II),

in welchen

R für einen gesättigten oder ungesättigten aliphatischen Kohlenwasserstoffrest mit 4 bis 17 Kohlenstoffatomen steht, der ein in (II) direkt an die Formamidgruppe bzw. in (I) direkt an die Nitrilgruppe gebundenes tertiäres Kohlenstoffatom enthält,

dadurch gekennzeichnet, dass man die N-substituierten Formamide (II) mit der mindestens stöchiometrischen Menge eines Carbonsäureanhydrids und/oder Keten in der Gasphase bei Temperaturen oberhalb von 250°C ohne Verwendung eines Katalysators umsetzt.

2. Verfahren nach Anspruch 1, dadurch gekennzeichnet, dass man bei Temperaturen zwischen etwa 320 und 500°C arbeitet.

3. Verfahren gemäss Anspruch 1, dadurch gekennzeichnet, dass man die N-substituierten Formamide (II) zunächst bei Temperaturen von etwa 390–420°C zu acylierten Formamiden umsetzt und diese anschliessend in einem höheren Temperaturbereich von etwa 420–500°C in Nitrile (I) und Carbonsäure fragmentieren lässt.

4. Verfahren nach Anspruch 1, dadurch gekennzeichnet, dass man auf 1 Mol N-substituiertes Formamid (II) 1,5–3 Mol Carbonsäureanhydrid bzw. Keten zum Einsatz bringt.

5. Verfahren nach Anspruch 1, dadurch gekennzeichnet, dass als Carbonsäureanhydrid Acetanhydrid eingesetzt wird.

6. Verfahren nach Anspruch 1, dadurch gekennzeichnet, dass als Carbonsäureanhydrid das Anhydrid der Säure eingesetzt wird, die dem gewünschten Nitril entspricht.

7. Verfahren nach Anspruch 1, dadurch gekennzeichnet, dass als N-substituiertes Formamid N-tert.-Butylformamid eingesetzt wird.

## Claims

1. Process for the preparation of nitriles having a tertiary alpha-C-atom of the general formula R-CN (I) from N-substituted formamides of the general formula

R-NH-CHO (II),

in which

R represents a saturated or unsaturated aliphatic hydrocarbon radical with 4 to 17 carbon atoms, which contains a tertiary carbon atom which is bonded in (II) directly to the formamide group or in (I) directly to the nitrile group,

characterised in that the N-substituted formamides (II) are reacted with an at least stoichiometric quantity of a carboxylic acid anhydride and/or ketene in the gas phase at temperatures above 250°C, without using a catalyst.

2. Process according to Claim 1, characterised in that it is carried out at temperatures between about 320 and 500°C.

3. Process according to Claim 1, characterised in that the N-substituted formamides (II) are first reacted at temperatures of about 390–420°C to give acylated formamides and these are then broken down in a higher temperature range of about 420–500°C to give nitriles (I) and carboxylic acid.

4. Process according to Claim 1, characterised in that 1.5–3 mols of the carboxylic acid anhydride or ketene are used per mol of N-substituted formamide (II).

5. Process according to Claim 1, characterised in that acetic anhydride is used as the carboxylic acid anhydride.

6. Process according to Claim 1, characterised in that the carboxylic acid anhydride used is the anhydride of the acid corresponding to the desired nitrile.

7. Process according to Claim 1, characterised in that N-tert.-butylformamide is used as the N-substituted formamide.

## Revendications

1. Procédé de préparation de nitriles comportant un atome de C-alpha tertiaire, de formule générale R-CN (I), à partir de formamides substitués à l'azote, de formule générale:

R-NH-CHO (II)

dans laquelle

R représente un radical d'hydrocarbure aliphatique saturé ou insaturé comportant 4 à 17 atomes de carbone, qui contient un atome de carbone tertiaire lié directement au groupe formamide en (II) ou lié directement au groupe nitrile en (I),

procédé caractérisé en ce qu'on fait réagir les formamides (II), substitués à l'azote, au moins avec la quantité stœchiométrique d'un anhydride d'acide carbocylique et/ou de cétène en phase gazeuse à des températures supérieures à 250°C sans utilisation d'un catalyseur.

2. Procédé selon la revendication 1, caractérisé en ce qu'on travaille à des températures comprises entre environ 320 et 500°C.

3. Procédé selon la revendication 1, caractérisé en ce qu'on fait réagir les formamides (II), substitués à l'azote, tout d'abord à des températures d'environ 390 à 420°C pour obtenir des formamides acylés et on laisse ceux-ci ensuite se fragmenter dans une gamme plus élevée de température d'environ 420 à 500°C en des nitriles (I) et de l'acide carboxylique.

4. Procédé selon la revendication 1, caractérisé en ce que l'on fait réagir pour 1 mole de formamide (II) substitué à l'azote 1,5 à 3 moles d'anhydride d'acide carboxylique ou de cétène.

5. Procédé selon la revendication 1, caractérisé en ce qu'on utilise comme anhydride d'acide carboxylique l'anhydride acétique.

6. Procédé selon la revendication 1, caractérisé en ce qu'on utilise comme anhydride d'acide carboxylique l'anhydride de l'acide correspondant au nitrile voulu.

7. Procédé selon la revendication 1, caractérisé en ce qu'on utilse comme formamide substitué à l'azote du N-tertiobutyl-formamide.